(19) Europäisches Patentamt
European Patent Office
Office européen des brevets

(11) **EP 3 027 053 B1**

(12) **FASCICULE DE BREVET EUROPEEN**

(45) Date de publication et mention
de la délivrance du brevet:
**02.01.2019 Bulletin 2019/01**

(21) Numéro de dépôt: **14790145.8**

(22) Date de dépôt: **17.07.2014**

(51) Int Cl.:
**A23L 29/00** (2016.01)     **A23L 17/60** (2016.01)
**A61K 36/05** (2006.01)

(86) Numéro de dépôt international:
**PCT/FR2014/051841**

(87) Numéro de publication internationale:
**WO 2015/007999 (22.01.2015 Gazette 2015/03)**

(54) **FARINE DE MICROALGUES RICHES EN LIPIDES ET PROCEDE DE PREPARATION**

LIPIDREICHES MIKROALGENMEHL UND VERFAHREN ZUR HERSTELLUNG DAVON

LIPID-RICH MICROALGAL FLOUR AND METHOD FOR PREPARING SAME

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **19.07.2013 FR 1357110**

(43) Date de publication de la demande:
**08.06.2016 Bulletin 2016/23**

(73) Titulaire: **Corbion Biotech, Inc.
South San Francisco, CA 94080 (US)**

(72) Inventeur: **PASSE, Damien
F-59500 Douai (FR)**

(74) Mandataire: **Cabinet Becker et Associés
25, rue Louis le Grand
75002 Paris (FR)**

(56) Documents cités:
**DE-A1-102006 056 454     US-A1- 2011 256 282
US-B1- 6 255 505     US-B1- 6 372 460
US-B2- 6 607 900**

- **Anonymous: "Solazyme Roquette Nutritionals
  Golden Chlorella Omega to be key ingredient in
  Natural Vitality Release of new 30oz Bottle for
  Energy28 Golden", , 10 mars 2011 (2011-03-10),
  XP055110155, Extrait de l'Internet:
  URL:http://investors.solazyme.com/common/d
  ownload/download.cfm?companyid=ABEA-673
  WYL
  &fileid=480217&filekey=31f6d18b-7dff-4396-
  8649-33dd2e6d4433&filename=588870.pdf
  [extrait le 2014-03-26]**
- **Elaine Watson: "Solazyme Breaking News on
  Food & Beverage Development -North America
  SPECIAL EDITION: PROTEIN-RICH FOODS...
  THE NEXT GENERATION Could algae be the next
  big thing in the protein market? Part one:
  Solazyme Roquette Nutritionals", , 23 janvier
  2013 (2013-01-23), XP055110159, Extrait de
  l'Internet:
  URL:http://www.foodnavigator-usa.com/conte
  nt/view/print/733996 [extrait le 2014-03-26]**

**Description**

La présente invention est relative à une farine de microalgues riches en lipides, microalgues du genre *Chlorella,* de préférence *Chlorella protothecoides.*

Plus particulièrement, la présente invention est relative à une farine de microalgues riches en lipides présentant - pour une distribution granulométrique, des valeurs de compression donnés - des propriétés d'écoulement, de mouillabilité et de dispersibilité dans l'eau tout à fait remarquables.

*Présentation de l'état de* **l'art**

Il existe plusieurs espèces d'algues qui peuvent être utilisées en Alimentaire, la plupart étant des « macroalgues » comme le varech, la laitue de mer (*Ulva lactuca*) et les algues rouges alimentaires de type *Porphyra* (cultivées au Japon) ou « dulse » (algue rouge *Palmaria palmata*).

Mais à côté de ces macroalgues, on trouve également de nombreuses sources d'algues que sont les « microalgues », i.e. algues microscopiques unicellulaires, photosynthétiques ou non, d'origine marine ou non, cultivées pour leurs applications en Biocarburant, Alimentaire, Cosmétique ou Nutrition - Santé.

Par exemple, la spiruline (*Arthrospira platensis*) est cultivée dans des lagunes ouvertes (en phototrophie) pour une utilisation comme complément alimentaire ou incorporée en petites quantités dans des confiseries ou des boissons (généralement moins de 0,5 % p/p).

D'autres microalgues riches en lipides, y compris certaines espèces de *Chlorella,* sont également très populaires dans les pays asiatiques comme compléments alimentaires (citons les microalgues du genre *Crypthecodinium* ou *Schizochytrium*).

La fraction huile de la biomasse de *Chlorella,* qui se compose essentiellement d'huiles monoinsaturées, fournit ainsi des avantages nutritionnel et santé par rapport aux huiles saturées, hydrogénées et polyinsaturées souvent trouvées dans les produits alimentaires conventionnels.

Les chlorelles sont donc exploitées en alimentation humaine ou animale, soit sous la forme de biomasse entière, soit sous la forme de farine, obtenu par séchage de biomasse de chlorelles dont la paroi cellulaire a été rompue par des moyens notamment mécaniques.

La farine de microalgues fournit également d'autres bénéfices, comme des micronutriments, des fibres alimentaires (glucides solubles et insolubles), des phospholipides, des glycoprotéines, des phytostérols, tocophérols, tocotriénols, et du sélénium.

Pour préparer la biomasse qui entrera dans la composition des aliments, la biomasse est concentrée, ou récoltée, du milieu de culture (culture réalisée par autotrophie à la lumière en photobioréacteurs, ou en hétérotrophie, à l'obscurité en présence d'une source carbonée assimilable par les Chlorelles).

Dans le domaine technique auquel s'adresse l'invention, la croissance des Chlorelles par voie hétérotrophique est préférée (voie dite fermentaire).

Au moment de la récolte de la biomasse de microalgues du milieu de fermentation, la biomasse comprend des cellules intactes pour l'essentiel en suspension dans un milieu de culture aqueux.

Pour concentrer la biomasse, on procède alors à une étape de séparation solide-liquide, par filtration frontale ou tangentielle, ou par centrifugation, par tout moyen connu par ailleurs de l'homme du métier.

Après concentration, la biomasse de microalgues peut être directement traitée afin de produire des gâteaux emballés sous vide, des paillettes d'algues, des homogénats d'algues, de la poudre d'algues intactes, de la farine d'algues broyées, ou de l'huile d'algues.

On procède également au séchage de la biomasse de microalgues pour faciliter le traitement ultérieur ou pour une utilisation de la biomasse dans ses différentes applications, notamment alimentaires.

Différentes textures et saveurs peuvent être conférées à des produits alimentaires, selon que la biomasse algale est séchée, et si oui, en fonction de la méthode de séchage mise en oeuvre.

Par exemple, le brevet US 6.607.900 décrit le séchage de la biomasse de microalgues en utilisant un séchoir à tambour sans centrifugation préalable, pour préparer des flocons (« flakes ») de microalgues.

De la poudre de microalgues peut être préparée à partir de la biomasse de microalgues concentrées à l'aide d'un sécheur pneumatique ou par atomisation, comme décrit dans le brevet US 6.372.460.

Dans un atomiseur, une suspension liquide est alors pulvérisée sous la forme d'une dispersion de fines gouttelettes dans un courant d'air chauffé. Le matériel entraîné est rapidement séché et forme une poudre sèche.

Dans d'autres cas, une combinaison d'un séchage par atomisation suivi de l'utilisation d'un séchoir à lit fluidisé est mise en oeuvre pour atteindre les conditions améliorées d'obtention d'une biomasse microalgale séchée (voir, par exemple, le brevet US 6.255.505).

Dans le domaine technique auquel s'adresse l'invention, on recherche plus particulièrement à préparer une farine d'algues produites par voie fermentaire.

Cette farine de microalgues au sens de l'invention est préparée à partir de la biomasse de microalgues concentrée qui a été mécaniquement lysée et homogénéisée, l'homogénat étant ensuite atomisé ou flash-séché.

La production de farine d'algues nécessite que les cellules soient lysées pour libérer leur huile.

Par exemple, un disrupteur à pression peut être utilisé pour pomper une suspension contenant les cellules à travers un orifice restreint pour lyser les cellules.

Une pression élevée (jusqu'à 1500 bar) est appliquée, suivie d'une expansion instantanée à travers une buse.

Le cassage des cellules peut être réalisé par trois mécanismes différents : empiètement sur la vanne, cisaillement élevé du liquide dans l'orifice, et chute de pression soudaine en sortie, provoquant une explosion de la cellule.

La méthode libère des molécules intracellulaires.

Un homogénéisateur NIRO Homogenizer Niro (GEA NIRO SOAVI - ou tout autre homogénéisateur haute pression) peut être utilisé pour traiter des cellules présentant une taille majoritairement comprise entre 0,2 et 5 microns.

Ce traitement de la biomasse algale sous haute pression (environ 1000 bar) lyse généralement plus de 90 % des cellules et réduit la taille à moins de 5 microns.

De manière alternative, un broyeur à billes est plutôt utilisé.

Dans un broyeur à billes, les cellules sont agitées en suspension avec de petites particules sphériques. Le cassage des cellules est provoqué par les forces de cisaillement, le broyage entre les billes, et les collisions avec des billes.

Ces billes cassent les cellules pour en libérer le contenu cellulaire. La description d'un broyeur à billes approprié est par exemple faite dans le brevet US 5.330.913.

On obtient une suspension de particules de plus petite taille que les cellules d'origine sous la forme d'une émulsion « huile dans eau ».

Cette émulsion est ensuite atomisée et l'eau est éliminée, laissant une poudre sèche contenant les débris cellulaires, du liquide intracellulaire et de l'huile.

Un exemple de procédé de production d'une farine de microalgues riche en lipides, séchée par atomisation standard, est décrit dans la demande US 2011/256282

Cependant, renfermant de l'huile à une teneur de 10, 25 voire 50 % en poids de la poudre sèche, on peut déplorer l'obtention d'une poudre sèche d'aspect adhésive et cohésive, qui s'écoule difficilement.

Les hautes teneurs en lipides (plus de 60 %) sont même considérées comme plus difficiles, voire impossible à sécher de façon efficace.

Il est également déploré des problèmes de mouillabilité et de dispersibilité dans l'eau des farines de biomasse séchées, présentant alors de moins bonnes propriétés de mouillabilité.

Pour résoudre les difficultés inhérentes au séchage de ces émulsions riches en lipides, l'homme du métier s'oriente généralement sur deux principales voies :

- le choix de dispositifs de séchage adaptés aux poudres riches en matière grasse, avec une mise en oeuvre particulière ;
- l'utilisation de différents agents d'écoulement (par exemple les produits dérivés de la silice) ou supports d'atomisation,
- la formulation (par encapsulation)

sans que ces deux voies ne soient mutuellement exclusives.

*Dispositifs d'atomisation*

Il existe de multiples dispositifs de séchage de composés riches en lipides par atomisation dans l'état de l'art. On peut trouver aisément dans la littérature des illustrations des technologies et appareillages proposés : par exemple dans le Spray Drying Handbook, de K. MASTERS, notamment dans sa 5ème édition, publiée en 1991 et republiée en 1994 par Longman Scientific & Technical (disponible à la British Library ou à la Library of Congress sous ISBN 0-470-21743-X) ou dans le BETE® Spray Dry Manual, 2005 (accessible à l'adresse internet www.bete.com)

Il apparait ainsi à la lecture de ces documents qu'aucune des solutions proposées n'est totalement satisfaisante, par exemple :

- pour sécher de produits lactés enrichis en matières grasses (20-30 %), il est classiquement utilisé des tours d'atomisation à co-courants munis de buses de vaporisation dans un dispositif en deux étages (le second dédié au conditionnement et refroidissement de la poudre obtenue dans le premier étage).

Cependant, des dépôts se forment facilement et augmentent le risque de prise de feu par des mécanismes d'auto-oxydation. Ce qui nécessite l'ajout de multiples systèmes d'extinction d'incendie.

- pour sécher du fromage fondu, le fromage est broyé et mélangé avec de l'eau pour former une crème onctueuse avant atomisation. L'atomisation est ensuite réalisée dans un atomiseur à fond conique.

Cependant, une fois encore en raison de la haute teneur en matière grasse, des dépôts se forment.

La solution proposée est d'utiliser des atomiseurs équipés de lits fluidisés ou de bandes mobiles intégrés dans la base de la chambre de l'atomiseur.

Cependant, demeurent toujours des problèmes de transport pneumatique de la poudre séchée ainsi obtenue.

- pour sécher des crèmes glacées non lactées, dans lesquelles des matières grasse végétales remplacent le beurre, et les caséinates de sodium remplacent les solides non gras du lait, la difficulté d'atomisation est due à la haute teneur en sucres (classiquement supérieure à 30 %).

Il est alors nécessaire d'atomiser en présence d'une partie du sucre, et de compléter la formule en ajoutant du sucre en poudre au mélange sec.

Par ailleurs, pour remédier aux problèmes de haute teneur en sucres, il faut pouvoir contrôler la production de poussières, gérer le transport pneumatique de la poudre, limiter son agglomération et éviter les dépôts à l'intérieur de la chambre d'atomisation.

Cependant, seules des solutions de séchage en chambre d'atomisation munie d'un lit fluidisé sont proposées.

- pour sécher des produits présentant de 35 à 80 % de matières grasses, le problème à éviter est la rupture des membranes protectives des globules lipidiques (notamment les protéines) ce qui conduit à un relargage desdits matières grasses durant le séchage.

La solution préconisée est l'augmentation du point de fusion des lipides, la formulation ou l'intégration dans l'atomiseur d'un système de lit de refroidissement à la base de la chambre d'atomisation.

Ou prévoir que l'air introduit à la base de ladite chambre d'atomisation soit refroidi par un air secondaire pour empêcher la fusion de la poudre dans la chambre, et solidifier la surface des particules avant toute manipulation mécanique.

Ou encore, si des cyclones sont prévus pour collecter la poudre, il faut introduire de l'air froid avant toute collecte, afin d'empêcher la fusion à l'intérieur des cyclones.

Finalement, il est préféré de mettre en oeuvre une configuration complexe, combinant une tour d'atomisation à buses avec un lit fluidisé intégré ou une bande mobile.

- pour le séchage d'algues,

Il est surtout décrit dans la littérature, comme décrit ci-avant, le séchage de microalgues entières, microalgues des genres *Chlorella* et *Spirulina.*

Leur forme poudre est alors destinée à faire des comprimés pour la préparation de compléments alimentaires en diététique.

L'atomisation est alors réalisée sur des biomasses de faible matière sèche (10 - 15 %) dans un atomiseur à turbine muni d'une chambre d'atomisation à cycle ouvert concourant.

En raison de cette faible matière sèche, seule une poudre de fine granulométrie est alors produite.

*Adjuvants de séchage*

Dans le domaine des succédanés de lait (termes anglais de « coffee/tea whiteners), il est question de compositions associant du caséinate de sodium, du sirop de maïs, des matières grasses végétales avec des agents émulsifiants, du phosphate de potassium et des silicates de sodium et d'aluminium.

L'atomisation est effectuée dans des atomiseurs à deux étages concourants munis de lits fluidisés vibrants externes.

Des particules de fine granulométrie sont produites.

Pour obtenir des particules agglomérées, on choisit des sécheurs où le lit fluidisé et la bande mobile est intégré(e).

Dans le domaine des huiles végétales, le séchage d'huile d'olive nécessite par ailleurs l'utilisation de supports d'atomisation, comme les maltodextrines.

***Objet de l'invention***

Il existe donc encore un besoin non satisfait pour de nouvelles formes stabilisées de farine de biomasse de microalgues riches en lipides afin de permettre leur incorporation aisée, à grande échelle, dans des produits alimentaires qui doivent rester savoureux et nutritifs.

La société Demanderesse a donc trouvé que ce besoin pouvait être satisfait en proposant une farine de microalgues riches en lipides présentant - pour une distribution granulométrique et des valeurs de compression donnés - des propriétés d'écoulement, de mouillabilité et de dispersibilité dans l'eau tout à fait remarquables.

En d'autres termes, la farine de microalgues riches en lipides de l'invention présente une granulométrie et des propriétés de compression équivalentes à une farine de microalgues riches en lipides standard, mais associées à des propriétés d'écoulement, de mouillabilité et de dispersibilité dans l'eau remarquables.

La farine de microalgues selon l'invention, qui comprend au moins 10% en poids sec de lipides, dont la taille de particules de farine est comprise entre 30 et 150 $\mu$m de diamètre et de compressibilité, mesurée sur POWDER TESTER HOSOKAWA, comprise entre 45 et 55 %, est alors caractérisée en ce qu'elle présente :

- une valeur d'écoulement, déterminées selon un test A décrit ci-après, comprise entre 55 et 60 % en poids pour le refus à 2 000 $\mu$m,
- une dispersibilité et une mouillabilité, exprimées selon un test B décrit ci-après, par :

  - la hauteur du produit décanté dans un bêcher, à une valeur comprise entre 0 et 2 mm ;
  - un degré d'humidification à une valeur de plus de 70 %, de préférence de plus de 80 % de la poudre totale.

De préférence, les microalgues sont du genre *Chlorella,* de préférence *Chlorella protothecoides.*

En outre, la farine de microalgues, et en particulier la biomasse de microalgues, peut comprendre au moins 20, 30, 40, 50 ou 60 % en poids sec de lipides.

La farine de microalgues selon l'invention est préparée par un procédé utilisant la technologie de séchage sur atomiseur « à fond plat » (terme anglo-saxon : Flat Bottom Spray Dryer ») couplé à un dispositif de balayage de la chambre d'atomisation avec de l'air à basse pression (terme anglo-saxon de « Air Broom »).

Comme il sera démontré ci-après, la conduite de ce séchage est particulièrement travaillée pour obtenir la farine de l'invention, en ce qui concerne :

- le rapport entre le débit d'air de séchage principal de l'atomiseur à fond plat (Flat Bottom Spray Dryer) et le débit d'air du dispositif de balayage (Air Broom),
- la température de l'air du dispositif de balayage (Air Broom).

Ainsi la présente invention est relative à un procédé de préparation de la farine de microalgues selon la présente invention, caractérisé en ce qu'il comprend :

1) préparer une émulsion de farine de microalgues riche en lipides dans de l'eau à une matière sèche comprise entre 15 et 50 % en poids sec, la farine de microalgues riche en lipides comprenant au moins 10 % en poids sec de lipides,

2) introduire cette émulsion dans un homogénéisateur haute pression,

3) pulvériser cette émulsion dans un atomiseur à fond plat (Flat Bottom Spray Dryer) muni d'un dispositif de balayage de la chambre d'atomisation avec de l'air à basse pression (Air Broom) à sa partie inférieure, tout en réglant de manière à ce que:

  a) la température de l'air de séchage principal soit comprise entre 160 et 240°C,

  b) la température de l'air dans la partie du dispositif de balayage soit d'au plus 70°C, de préférence d'au plus 65°C, plus préférentiellement comprise entre 50 et 60°C,

  c) le rapport entre le débit d'air du dispositif de balayage sur le débit d'air de séchage principal soit d'une valeur supérieure à 1/3, de préférence comprise entre 1/3 à 1/2,

  d) la température de l'air de refroidissement soit comprise entre 25 et 35°C, de manière à ce que la farine en sortie de l'atomiseur présente une température comprise entre 60° et 90°C.

4) collecter la farine de microalgues ainsi obtenue.

De préférence, les microalgues sont du genre genre *Chlorella,* de préférence *Chlorella protothecoides.* En outre, les microalgues, et en particulier la biomasse de microalgues, comprend au moins 10, 20, 30, 40, 50 ou 60 % en poids sec de lipides.

La présente invention est en outre relative à l'utilisation de la farine selon la présente invention dans les domaines alimentaires. Notamment, elle est relative à une méthode de préparation d'une composition alimentaire comprenant l'ajout d'une telle farine de microalgues à des ingrédients de la composition alimentaire.

### Description détaillée de l'invention

La présente invention est ainsi relative à la biomasse de microalgues propre à la consommation humaine qui est riche en nutriments, notamment en lipides.

L'invention se rapporte plus particulièrement à une farine de microalgues qui peut être incorporée dans des produits alimentaires dans lesquels la teneur en huiles de la farine de microalgues peut permettre une substitution en totalité ou partie des huiles et / ou graisses présentes dans les produits alimentaires conventionnels.

Au sens de l'invention, on entend par « farine de microalgues » le produit séché de la rupture des parois cellulaires de la biomasse de microalgues, notamment par des moyens mécaniques.

Au sens de l'invention, les microalgues considérées sont les espèces qui produisent des huiles de triglycérides appropriées et / ou des lipides totaux.

La biomasse de microalgues comprend au moins 10 % en poids sec d'huiles ou de lipides, de préférence au moins 25 à 35 % ou plus en poids sec d'huiles ou de lipides.

Plus préférentiellement encore, la biomasse contient au moins 40 %, au moins 50 %, au moins 75 % en poids sec d'huiles ou de lipides.

Les microalgues préférées de l'invention peuvent croître en conditions hétérotrophiques (sur sucres comme source carbonée et en l'absence de lumière).

La société Demanderesse recommande de choisir des microalgues du genre *Chlorella* riches en lipides.

Les microalgues utilisées peuvent être choisies, et de manière non-exhaustive, parmi les *Chlorella protothecoides, Chlorella kessleri, Chlorella minutissima, Chlorella sp., Chlorella sorokiniama, Chlorella luteoviridis, Chlorella vulgaris, Chlorella reisiglii, Chlorella ellipsoidea, Chlorella saccarophila, Parachlorella kessleri, Parachlorella beijerinkii, Prototheca stagnora et Prototheca moriformis.* De manière préférée, les microalgues utilisées selon l'invention appartiennent à l'espèce *Chlorella protothecoides.*

Les microalgues sont cultivées en milieu liquide pour produire la biomasse en tant que telle.

Selon l'invention, les microalgues sont cultivées dans un milieu contenant une source de carbone et une source d'azote en absence de lumière (conditions hétérotrophiques).

Les milieux de croissance solides et liquides sont généralement disponibles dans la littérature, et les recommandations pour la préparation des milieux particuliers qui conviennent à une grande variété de souches de microorganismes peuvent être trouvées, par exemple, en ligne à www.utex.org/, un site maintenu par l'Université du Texas à Austin pour sa collection de culture d'algues (UTEX).

La production de biomasse est réalisée en fermenteurs (ou bioréacteurs).

Les exemples spécifiques de bioréacteurs, les conditions de culture, et la croissance hétérotrophe et les méthodes de propagation peuvent être combinés de toute manière appropriée pour améliorer l'efficacité de la croissance microalgale et des lipides.

Dans le domaine technique auquel s'adresse l'invention, on recherche à préparer une farine d'algues.

Cette farine de microalgues au sens de l'invention est préparée à partir de la biomasse de microalgues concentrée qui a été mécaniquement lysée et homogénéisée, l'homogénat étant ensuite séché.

La farine de microalgues selon l'invention, de taille de particules comprise entre 30 et 150 $\mu$m et de compressibilité, mesurée sur POWDER TESTER HOSOKAWA, comprise entre 45 et 55 %, est caractérisée en ce qu'elle présente :

- une valeur d'écoulement, déterminées selon un test A, comprise entre 55 et 60 % en poids pour le refus à 2 000 $\mu$m,
- une dispersibilité et une mouillabilité, exprimées selon un test B, par :

  - la hauteur du produit décanté dans un bêcher, à une valeur comprise entre 0 et 2 mm ;
  - un degré d'humidification à une valeur de plus de 70 %, de préférence de plus de 80 % de la poudre totale.

Dans le domaine technique auquel s'attache l'invention, la farine de microalgues présente des paramètres de granulométrie et de compression communément retrouvés pour les farines de microalgues de Chlorella séchées par voie classique (atomisation simple effet) :

- La farine de microalgues selon l'invention présente une taille de particules comprise entre 30 et 150 $\mu$m.

Cette mesure est réalisée sur un granulomètre laser LS de marque COULTER®, muni de son module de dispersion petit volume ou SVM (125 ml) en suivant les spécifications du constructeur (dans les *« Small Volume Module Operating instructions »*).

- La farine de microalgues selon l'invention présente une compressibilité, mesurée sur POWDER TESTER HOSOKAWA, comprise entre 45 et 55 %.

La valeur de compression ou compressibilité C est obtenue par le calcul du rapport des valeurs de densité aérée (« Aerated Bulk Density » = A) et densité tassée (« Packed Density » = B), elles-mêmes déterminée par l'utilisation d'un appareil commercialisé par la société HOSOKAWA sous la marque POWDER TESTER type PTE en appliquant la méthode recommandée dans les instructions d'utilisation (« operating Instructions ») pour mesurer les densités aérée et tassée, selon l'équation suivante :

$$C = \frac{100\,(B-A)}{B}$$

A titre comparatif, la valeur de compression d'une farine de microalgue séchée en atomisation simple effet est de l'ordre de 47%

Cependant, et de manière surprenante et inattendue, la farine de microalgues conforme à l'invention est caractérisée par ses remarquables propriétés d'écoulement, de mouillabilité et dispersibilité.

- La farine de microalgues selon l'invention présente des propriétés d'écoulement, mesurée selon un test A, meilleures que celles mesurées pour une farine de microalgues séchées par voie classique.

Le test A consiste à mesurer le degré de cohésion de la farine de microalgues.

Ce test de cohésion est inspiré du test de cohésion décrit dans les instructions d'utilisation (« Operating Instructions ») du Powder Characteristics Tester type PTE commercialisé par la société HOSOKAWA.

Le test A consiste tout d'abord à tamiser la farine de microalgues selon l'invention sur un tamis d'ouverture de maille de 800 $\mu$m.

La fraction de la farine présentant une taille inférieure à 800 $\mu$m est ensuite récupérée, introduite dans un récipient fermé et subit un mélange par mouvement épicycloïdal à l'aide d'un mélangeur de laboratoire de marque TURBULA type T2C.

Par ce mélange, selon ses propres caractéristiques, la farine de microalgues conforme à l'invention exprimera sa propension à s'agglomérer ou à se repousser.

La farine ainsi mélangée est ensuite déposée sur un tamis de 2000 $\mu$m pour un nouveau tamisage.

Le tamisage terminé, le refus sur ce tamis est quantifié et le résultat donne une illustration du caractère « cohésif » ou « collant » de la farine de microalgues.

Ainsi, une poudre à écoulement libre, donc peu cohésive, ne sera pratiquement pas arrêtée par ce tamis de grande ouverture.

Le protocole est le suivant :

◦ tamiser la quantité de produit nécessaire sur un tamis de 800 $\mu$m pour récupérer 50 g de produit de taille inférieure à 800 $\mu$m,
◦ introduire ces 50 g de farine de taille inférieure à 800 $\mu$m dans un bocal en verre de contenance de 1 litre (Ref. BVBL Verrerie Villeurbannaise-Villeurbanne France) et refermer le couvercle,
◦ disposer ce bocal dans le mélangeur (TURBULA modèle T2C) réglé sur la vitesse de 42 t/mn (Willy A. Bachofen Sarl-Sausheim-France) et mélanger pendant 5 minutes,
◦ préparer le tamis (de marque Saulas -Diamètre 200 mm ; Paisy Cosdon - France) qui sera placé sur un tamiseur (de marque Fritsch modèle Pulverisette type 00.502) ; détail du montage en partant du bas vers le haut : tamiseur, fond de tamis, tamis de 800 $\mu$m, tamis de 2000 $\mu$m, couvercle du tamiseur.
◦ déposer la poudre issue du mélange sur le haut de la colonne (tamis de 2000 $\mu$m), fermer avec le couvercle du tamiseur et tamiser pendant 5 minutes sur le tamiseur (FRITSCH), avec une amplitude 5 en position permanente,
◦ peser le refus sur ce tamis.

La farine de microalgues présente alors entre 55 et 60 % en poids pour le refus à 2 000 $\mu$m.

A titre comparatif, la valeur d'écoulement d'une farine de microalgue classique est de l'ordre de 71 %.

- La farine de microalgues selon l'invention présente des propriétés de dispersibilité et de mouillabilité tout à fait remarquables.

Ces dispersibilité et mouillabilité sont exprimées selon un test B, par :

  ◦ la hauteur du produit décanté dans un bêcher, à une valeur comprise entre 0 et 2 mm ;
  ◦ un degré d'humidification à une valeur de plus de 70 %, de préférence de plus de 80 % de la poudre totale.

Ce caractère surprenant et inattendu repose sur le fait que les mesures de compressibilité et d'écoulement démontrent que la farine de microalgues conforme à l'invention reste, tout comme les farines de microalgues classiques, assez cohésive, puisqu'après mélange, mettant en oeuvre peu d'énergie mécanique (durée de tamisage d'à peine 5 mn), encore 55 à 60 % des particules inférieures à 800 μm ne parviennent pas à traverser un tamis de 2000 μm, d'une ouverture pourtant 2 à 4 fois plus grande.

Il s'en déduit aisément qu'une telle farine, présentant un tel comportement, devrait être peu dispersible et ainsi être difficile à mettre en oeuvre dans une préparation où une répartition homogène des ingrédients est recommandée.

De la même façon, sa mouillabilité devrait être peu importante.

La mouillabilité est une propriété technologique très souvent utilisée pour caractériser une poudre remise en suspension dans l'eau, par exemple en Industries Laitières.

Elle traduit l'aptitude d'une poudre à s'immerger après avoir été déposée à la surface de l'eau (Haugaard Sorensen et al., « Méthodes d'analyse des produits laitiers déshydratés », Niro A/S (ed.), Copenhagen, Denmark, 1978), et reflète ainsi la capacité de la poudre à absorber de l'eau à sa surface (Cayot et Lorient, « Structures et technofonctions des protéines du lait ». Paris : Airlait Recherches: Tec et Doc, Lavoisier, 1998).

La mesure de cet indice consiste classiquement à mesurer le temps nécessaire à une certaine quantité de poudre pour pénétrer dans l'eau à travers sa surface libre au repos.

Selon Haugaard Sorensen et al. (1978) :

-   une poudre est dite « mouillable » si son « Indice de Mouillabilité » est inférieur à 20 secondes.
-   il faut également associer à la mouillabilité, l'aptitude au gonflement de la poudre. En effet, lorsqu'une poudre absorbe de l'eau, elle gonfle progressivement. Puis, la structure de la poudre disparaît lorsque les divers constituants sont solubilisés ou dispersés.

On exprimera cette aptitude au gonflement par un % de produit humidifié.

Parmi les facteurs influençant la mouillabilité, il y a la présence de grosses particules primaires, la réintroduction des fines, la masse volumique de la poudre, la porosité et la capillarité des particules de poudre ainsi que la présence d'air, la présence de matières grasses à la surface des particules de poudre et les conditions de reconstitution.

Le test B mis au point par la société Demanderesse consiste ici à considérer plus particulièrement :

-   le comportement de la poudre de farine de microalgues lorsqu'elle est mise en contact de l'eau, par la mesure, après un certain temps de contact, de la hauteur de la poudre qui décante lorsque la poudre est placée à la surface de l'eau.
-   sa capacité de reprise en eau (exprimée en %).

Le protocole de ce test est le suivant :

  ◦ dans un bêcher de forme basse de 600 ml (bêcher FISCHERBRAND FB 33114), introduire 500 ml d'eau déminéralisée à 20°C,
  ◦ placer uniformément 25 g de la poudre de farine de microalgues à la surface de l'eau, sans mélanger,
  ◦ observer le comportement de la poudre au cours du temps,
  ◦ mesurer la hauteur du produit décanté au fond du bêcher.

Une poudre très cohésive, de faible mouillabilité demeurera à la surface du liquide, tandis qu'une poudre de meilleure mouillabilité décantera plus aisément.

La farine de microalgues selon l'invention présente une dispersibilité et une mouillabilité exprimées selon le test B, par :

  ◦ la hauteur du produit décanté dans un bêcher, à une valeur comprise entre 0 et 2 mm ;
  ◦ un degré d'humidification à une valeur de plus de 70 %, de préférence de plus de 80 % de la poudre totale.

Or, à titre comparatif, la farine de microalgues séchées classiquement par atomisation simple effet se maintient à la surface de l'eau, et ne s'hydrate pas suffisamment pour pouvoir décanter au fond du bêcher.

La farine de microalgues conforme à l'invention est susceptible d'être obtenue par une conduite particulière d'un procédé de séchage par atomisation, séchage sur atomiseur « à fond plat » (terme anglo-saxon : « Flat Bottom Spray Dryer » ou FBSD) couplé à un dispositif de balayage de la chambre d'atomisation avec de l'air à basse pression (terme anglo-saxon de « Air Broom » ou AB).

L'atomiseur à fond plat « Flat Bottom Spray Dryer » est classiquement utilisé pour le séchage de matières riches en matières grasses ou des produits hygroscopiques, ou de manière plus prosaïque, dans des structures où l'on manque de place... Cependant, à la connaissance de la société Demanderesse, il n'a jamais été utilisé couplé à un dispositif de balayage de la chambre d'atomisation avec de l'air à basse pression « Air Broom » pour le séchage de biomasse lysée de microalgues en général et de *Chlorella* en particulier.

Quant au dispositif couplant FBSD et AB, il est surtout recommandé pour le séchage de fruits, pulpes végétales et jus de fruits, voire pour des extraits de viande.

Pour procéder au séchage de la biomasse de microalgues lysées, en suivant ce principe d'atomisation, on peut employer par exemple un atomiseur FBSD muni de l'AB commercialisé par les sociétés CE Rogers, Marriott Walker, Henningsen Foods ou Food Engineering Co. and Henszey Co.

De manière surprenante et inattendue, la société Demanderesse a ainsi constaté que le séchage de la farine de microalgues par la mise en oeuvre par exemple de ce procédé FBSD / AB, permettait non seulement de préparer avec un haut rendement un produit présentant un profil granulométrique, une aptitude à l'écoulement et une compressibilité standard, mais surtout de lui conférer des propriétés inattendues d'écoulement, de mouillabilité et de dispersibilité dans l'eau, sans qu'il ne soit utile d'utiliser d'agents liant de granulation, ni d'agents anti-mottant.

En effet, les procédés décrits antérieurement (telle l'atomisation simple effet qui est utilisée classiquement pour sécher des biomasses ou farines de microalgues) ne permettent pas d'obtenir l'ensemble des caractéristiques souhaitées.

Le procédé de préparation de la farine de microalgues conforme à l'invention comprend alors les étapes suivantes :

1) préparer une émulsion de farine de microalgues riche en lipides dans de l'eau à une matière sèche comprise entre 15 et 50 % en poids sec, la farine de microalgues riche en lipides comprenant au moins 10 % en poids sec de lipides,
2) introduire cette émulsion dans un homogénéisateur haute pression,
3) la pulvériser dans un atomiseur FBSD muni d'un dispositif AB à sa partie inférieure, de manière à ce que :

a) la température de l'air de séchage principal soit comprise entre 160 et 240°C,
b) la température de l'air dans la partie Air Broom soit d'au plus 70°C, de préférence d'au plus 65°C, plus préférentiellement comprise entre 50 et 60°C,
c) le rapport entre le débit d'air de l'AB sur le débit d'air de séchage principal soit d'une valeur supérieure à 1/3, de préférence comprise entre 1/3 à 1/2,
d) la température de l'air de refroidissement soit comprise entre 25 et 35°C, de manière à ce que la farine en sortie de l'atomiseur présente une température comprise entre 60° et 90°C.

4) collecter la farine de microalgues ainsi obtenue.

La première étape du procédé de l'invention consiste à préparer une émulsion de farine de microalgues riche en lipides dans de l'eau à une matière sèche comprise entre 15 et 50 % en poids sec. En particulier, la teneur en matière sèche peut être comprise entre 25 et 45 %, de préférence entre 35 et 45 %. En outre, la teneur en lipides de la farine de microalgues ou de la biomasse de microalgues est de préférence d'au minimum au moins 20, 30, 40, 50 ou 60 % en poids sec, par exemple entre 20 et 80 % ou 30 et 70 %. Facultativement, le taux de broyage de la biomasse de microalgues peut être au minimum de 25% à 75% de cellules lysées, par exemple de 50%, 85% ou 95 % de lyse des cellules, de préférence est de 85% ou 95 %.

Comme il sera exemplifié ci-après, la biomasse obtenue en fin de fermentation présente typiquement une teneur en lipides d'environ 50%, de 10 à 50 % en fibres, 2 à 15 % de protéines, 30 % de sucres et 10 % d'amidon.

La biomasse est ensuite :

- désactivée par traitement thermique flash (traitement HTST),
- lavée par dilution avec une solution aqueuse et concentration par centrifugation,
- broyée au broyeur à billes créant ainsi une émulsion « huile dans eau ».

La seconde étape du procédé de l'invention consiste à introduire cette émulsion dans un homogénéisateur haute pression.

La société Demanderesse recommande de procéder à cette homogénéisation de l'émulsion dans un dispositif à deux étages, par exemple un homogénéiseur GAULIN commercialisé par la société APV, avec une pression de 160 bars au premier étage, et 40 bar au deuxième étage.

La troisième étape du procédé de l'invention consiste à pulvériser cette solution dans un atomiseur FBSD muni d'un dispositif AB à sa partie inférieure de manière à ce que :

a) la température de l'air de séchage principal soit comprise entre 160 et 240°C,

b) la température de l'air dans la partie AB soit d'au plus 70°C, de préférence d'au plus 65°C, plus préférentiellement comprise entre 50 et 60°C,

c) le rapport entre le débit d'air de l'AB sur le débit d'air de séchage principal soit d'une valeur supérieure à 1/3, de préférence comprise entre 1/3 à 1/2

d) la température de l'air de refroidissement soit comprise entre 25 et 35°C, de manière à ce que la farine en sortie de l'atomiseur présente une température comprise entre 60° et 90°C.

C'est ainsi que, comme il sera exemplifié ci-après, pour un débit d'air de séchage principal fixé à 2200 kg/h, le débit d'air de l'AB sera de plus de 750 kg/h, de préférence compris entre 800 et 900 kg/h.

Comme il sera également exemplifié ci-après, ces conditions opératoires permettent de limiter la formation de dépôt sur les parois de la chambre d'atomisation et ainsi d'optimiser le rendement de séchage à plus de 90 %, de préférence à plus de 95 %, plus préférentiellement encore à 99 %.

Par ailleurs, ce sont ces paramètres de contrôle de l'AB, plus que la conduite d'atomisation sur le FBSD, qui permettent d'obtenir les produits présentant de telles propriétés de mouillabilité et dispersibilité dans l'eau.

La dernière étape du procédé conforme à l'invention consiste enfin à collecter la farine de microalgues ainsi obtenue.

Cette farine de microalgues est utile dans le domaine alimentaire. Ainsi la présente invention est relative à l'utilisation de la farine selon la présente invention ou obtenue par le procédé selon la présente invention dans les domaines alimentaires. Notamment, elle est relative à une méthode de préparation d'une composition alimentaire comprenant l'ajout d'une telle farine de microalgues à des ingrédients de la composition alimentaire ou à la composition alimentaire. De telles utilisations sont par exemple décrites dans les demandes de brevet WO2010/045368, WO2010/120923 ou US2010/0297296.

L'invention sera mieux comprise à l'aide des exemples qui suivent, lesquels se veulent illustratifs et non limitatifs.

## EXEMPLES

### Exemple 1. Obtention de la Biomasse de la microalgue *Chlorella protothecoides* par voie fermentaire

Le protocole de fermentation est adapté de celui décrit en toute généralité dans la demande de brevet WO 2010/120923.

Le fermenteur de production est inoculé avec une préculture de Chlorella protothecoides. Le volume après inoculation atteint 9 000 L.

La source de carbone employée est un sirop de glucose 55 % p/p stérilisé par application d'un barème temps/température.

La fermentation est un « fed-batch » durant lequel le débit de glucose est ajusté de sorte à maintenir une concentration de glucose résiduel de 3 à 10 g/L.

La durée du fermenteur de production est de 4 à 5 jours.

En fin de fermentation, la concentration cellulaire atteint 185 g/L.

Pendant la phase d'alimentation en glucose, la teneur en azote dans le milieu de culture est limitée pour permettre l'accumulation de lipides à hauteur de 50 %.

La température de fermentation est maintenue à 28°C.

Le pH de fermentation avant inoculation est ajusté à 6,8 puis est régulé sur cette même valeur pendant la fermentation.

L'oxygène dissous est maintenu à un minimum de 30 % en contrôlant l'aération, la contre pression et l'agitation du fermenteur.

Le moût de fermentation est traité thermiquement sur une zone HTST avec un barème de 1 min à 75°C et refroidi à 6°C.

La biomasse est alors lavée à l'eau potable décarbonatée avec un ratio de dilution de 6 pour 1 (eau/moût) et concentrée à 250 g/L (25% DCW (« *Dry Cell Weight* », poids sec en cellules) par centrifugation en utilisant une Alfa Laval FEUX 510.

Les cellules sont désactivées par traitement thermique à travers une zone HTST à 75°C pendant 1 minute.

Pour la suite des opérations, la température est maintenue sous 8-10°C.

La concentration en solubles interstitielles est réduite par lavage de la biomasse par dilution (3 : 1 ($V_{eau}$/$V_{biomasse}$)) et concentration par centrifugation (centrifugeuse à assiettes et buses).

Après cette étape, la matière sèche de la biomasse est d'environ 25% sortie séparatrice, puis concentrée à 45 % par évaporation.

La biomasse lavée est broyée à l'aide d'un broyeur à billes type « bead mill » avec un taux de broyage de 95 %.

L'émulsion grossière type « huile dans eau » ainsi générée est homogénéisée sous pression dans un homogénéisateur GAUVIN à deux étages (160 bars au 1er étage / 40 bars au second) après ajustement du pH à 7 à la potasse.

La biomasse obtenue en fin de fermentation présente typiquement une teneur en lipides d'environ 50%, de 10 à 50 % en fibres, 2 à 15 % de protéines, 30 % de sucres et 10 % d'amidon, les pourcentages étant exprimés en poids sec de biomasse totale.

**Exemple 2. Séchage de l'émulsion « huile dans eau » homogénéisée de farine de microalgues**

On procède au séchage de l'émulsion homogénéisée obtenue à l'exemple 1 :

- dans un atomiseur simple effet (liquide séché par un seul passage dans le flux de chaleur puis récupéré en bas de la tour au niveau du cyclone ou du filtre à manche), commercialisée par GEA NIRO, de manière à obtenir une farine de microalgues témoin, conforme à ce qui est commercialement accessible.

Ou

- dans un dispositif d'atomisation à fond plat (Flat Bottom Spray Dryer) muni d'un dispositif interne de balayage de la chambre d'atomisation avec de l'air à basse pression (Air Broom), pour obtenir la farine de microalgues conforme à l'invention,

Les conditions opératoires d'atomisation simple effet Témoin sont les suivantes :

- température d'entrée de 160°C,
- température de 60°C dans la section de séchage,
- température de refroidissement de l'air : 21°C
- température de sortie : 60°C.

Quant au procédé d'atomisation conforme à l'invention, il consiste à pulvériser la suspension homogénéisée à haute pression dans un dispositif d'atomisation à fond plat (Flat Bottom Dryer) muni d'un dispositif de balayage d'air (Air Broom) de la manière suivante :

- Système d'alimentation : cuve d'alimentation avec mélangeur à hélice et enveloppe chauffante ; mono pompe ; filtre Duplex
- Atomiseur : Centrifuge, 160 mm de diamètre,
- Déchargeur de poudres : dispositif rotatif pour éviter l'agglomération au fond de la chambre,
- Air de sortie : air chargée de particules quitte la chambre par le bas ; la poudre sèche est séparée de l'air dans un filtre à manche,

Les paramètres de conduite d'atomisation dans l'atomisation à fond plat (Flat Bottom Spray Dryer) sont les suivants :

- Alimentation : émulsion à 20 % de matière sèche et à une température de 50 - 65°C
- turbine à 16.400 rpm
- débit de l'émulsion : 60 kg/h - 160 kg/h
- Air principal :

  ◦ Débit : 2200 kg/h
  ◦ Température : 165 - 220 °C

Deux configurations de conduite de balayage d'air (Air Broom) sont réalisées :

1. conduite standard,
2. conduite optimisée pour générer la farine de microalgues conforme à l'invention

La conduite de balayage d'air (Air Broom) standard (selon les spécifications du constructeur) est la suivante (pour 1 essai) :

- débit d'air : 700 kg/h à une température de 70°C
- Température de sortie en bas de chambre : 95°C
- Air de refroidissement : 600 kg/h à une température de 30°C
- Température de l'air avant le filtre à manche : 81°C

Le rendement de production est ici < 90 %, et on constate la formation de dépôts sur les parois de la chambre d'atomisation.

La conduite de balayage d'air (Air Broom) optimisée par la société Demanderesse pour conduire à la farine de microalgues conforme à l'invention est la suivante (essai réalisé en triple) :

- débit d'air : 850 kg/h à une température de 65°C
- Air de refroidissement : 800 kg/h à une température de 30°C.

Le rendement de production est ici de 99 %, et on ne constate pas la formation de dépôts sur les parois de la chambre d'atomisation.

**Exemple 3. Caractérisation de la farine de microalgues conforme à l'invention**

Sont présentées dans le tableau suivant les valeurs des paramètres de :

- granulométrie,
- compressibilité,
- aptitude à l'écoulement par test de cohésion (2000 μm)
- mouillabilité,
- dispersibilité dans l'eau

pour le Témoin « atomisation simple effet », pour le Témoin « Air Broom » non optimisé et pour les trois lots de farine de microalgues conforme à l'invention.

| | Granulométrie (D 4,3 - μm) | Densité aérée (g/ml) | Densité tassée (g/ml) | Compressibilité (%) |
|---|---|---|---|---|
| Témoin Atomisation Simple Effet | 40 | 0,27 | 0,51 | 47 |
| Témoin Air Broom « non optimisé » | 133 | 0,35 | 0,45 | 22 |
| Lot 1 | 55 | 0,3 | 0,59 | 49 |
| Lot 2 | 90 | 0,26 | 0,54 | 52 |
| Lot 3 | 110 | 0,27 | 0,53 | 49 |

| | Cohésion 2 000 μm | Mouillabilité (mm) | Dispersibilité (% de produit humidifié après 2 minutes - valeur constante ensuite) |
|---|---|---|---|
| Témoin Atomisation Simple Effet | 71 | 0 Reste à la surface de l'eau | 0 |
| Témoin Air Broom « non optimisé » | 65 | 0 Pénétration dans l'eau ; pas de décantation | 0 |
| Lot 1 | 59 | 2 | 70 |

(suite)

|  | Cohésion 2 000 μm | Mouillabilité (mm) | Dispersibilité (% de produit humidifié après 2 minutes - valeur constante ensuite) |
|---|---|---|---|
| Lot 2 | 59 | 0 Pénétration dans l'eau ; pas de décantation | 80 |
| Lot 3 | 57 | 0 Pénétration dans l'eau ; pas de décantation | 80 |

Tout à fait logiquement, on constate que la farine de microalgues classique (Témoin « Atomisation Simple effet »), caractérisée par des particules « cohésives », ne parvient pas à s'hydrater suffisamment pour décanter, tandis que la farine de microalgues conforme à l'invention y parvient sans difficulté malgré son caractère cohésif. Ainsi, la farine de microalgues classique se dépose à la surface de l'eau sans y pénétrer. A contrario, la farine de microalgues conforme à l'invention s'hydrate bien et ne décante pas au fond du bêcher.

Quant à l'essai « Air Broom » standard, on constate que la poudre obtenue ne présente aucune mouillabilité ni dispersibilité dans l'eau.

**Revendications**

1. Farine de microalgues comprenant au moins 10% en poids sec de lipides, la taille de particules de farine étant comprise entre 30 et 150 μm de diamètre et de compressibilité, mesurée sur POWDER TESTER HOSOKAWA, comprise entre 45 et 55 %, **caractérisée en ce qu'**elle présente :

   - une valeur d'écoulement, déterminées selon le test A mentionné dans la description, comprise entre 55 et 60 % en poids pour le refus à 2 000 μm,
   - une dispersibilité et une mouillabilité, exprimées selon le test B mentionné dans la description, par :

      ■ la hauteur du produit décanté dans un bêcher, à une valeur comprise entre 0 et 2 mm ;

   un degré d'humidification à une valeur de plus de 70 %, de préférence de plus de 80 % de la poudre totale, et **en ce qu'**elle est préparée suivant un procédé comprenant les étapes consistant à :

      1) préparer une émulsion de farine de microalgues riche en lipides dans de l'eau à une matière sèche comprise entre 15 et 50 % en poids sec, la farine de microalgues riche en lipides comprenant au moins 10 % en poids sec de lipides,
      2) introduire cette émulsion dans un homogénéisateur haute pression,
      3) pulvériser cette émulsion dans un atomiseur à fond plat muni d'un dispositif de balayage de la chambre d'atomisation avec de l'air à basse pression à sa partie inférieure, tout en réglant de manière à ce que :

         a) la température de l'air de séchage principal soit comprise entre 160 et 240°C,
         b) la température de l'air dans la partie du dispositif de balayage soit d'au plus 70°C, de préférence d'au plus 65°C, plus préférentiellement comprise entre 50 et 60°C,
         c) le rapport entre le débit d'air du dispositif de balayage sur le débit d'air de séchage principal soit d'une valeur supérieure à 1/3, de préférence comprise entre 1/3 à 1/2,
         d) la température de l'air de refroidissement soit comprise entre 25 et 35°C, de manière à ce que la farine en sortie de l'atomiseur présente une température comprise entre 60° et 90°C,

      4) collecter la farine de microalgues ainsi obtenue.

2. Farine de microalgues selon la revendication 1, **caractérisée en ce que** les microalgues sont du genre genre *Chlorella,* de préférence *Chlorella protothecoides.*

3. Procédé de préparation de la farine de microalgues selon l'une quelconque des revendications 1 et 2, **caractérisé en ce qu'**il comprend :

1) préparer une émulsion de farine de microalgues riche en lipides dans de l'eau à une matière sèche comprise entre 15 et 50 % en poids sec, la farine de microalgues riche en lipides comprenant au moins 10 % en poids sec de lipides,

2) introduire cette émulsion dans un homogénéisateur haute pression,

3) pulvériser cette émulsion dans un atomiseur à fond plat muni d'un dispositif de balayage de la chambre d'atomisation avec de l'air à basse pression à sa partie inférieure, tout en réglant de manière à ce que :

a) la température de l'air de séchage principal soit comprise entre 160 et 240°C,

b) la température de l'air dans la partie du dispositif de balayage soit d'au plus 70°C, de préférence d'au plus 65°C, plus préférentiellement comprise entre 50 et 60°C,

c) le rapport entre le débit d'air du dispositif de balayage sur le débit d'air de séchage principal soit d'une valeur supérieure à 1/3, de préférence comprise entre 1/3 à 1/2,

d) la température de l'air de refroidissement soit comprise entre 25 et 35°C, de manière à ce que la farine en sortie de l'atomiseur présente une température comprise entre 60° et 90°C.

4) collecter la farine de microalgues ainsi obtenue.

4. Utilisation de la farine de l'une quelconque des revendications 1 et 2 dans les domaines alimentaires.

5. Méthode de préparation d'une composition alimentaire comprenant l'ajout d'une farine de microalgues selon l'une quelconque des revendications 1 et 2 à des ingrédients de la composition alimentaire.

**Patentansprüche**

1. Mikroalgenmehl umfassend wenigstens 10 Trockengew.-% Lipide, wobei die Größe der Mehlpartikel zwischen 30 und 150 $\mu$m im Durchmesser beträgt und die Kompressibilität, gemessen mit einem POWDER TESTER HOSOKAWA, zwischen 45 und 55 % beträgt, **dadurch gekennzeichnet, dass** es aufweist:

- einen Fließwert, bestimmt nach dem in der Beschreibung erwähnten Test A, zwischen 55 und 60 Gew.-% für den Siebrückstand bei 2000 $\mu$m,

- eine Dispergierbarkeit und eine Benetzbarkeit, ausgedrückt nach dem in der Beschreibung erwähnten Test B mithilfe:

• der Höhe des abgesetzten Produkts in einem Becherglas mit einem Wert zwischen 0 und 2 mm;

- einen Durchfeuchtungsgrad mit einem Wert von mehr als 70%, bevorzugt mehr als 80% des Gesamtpulvers, und **dadurch gekennzeichnet, dass** es nach einem Verfahren hergestellt ist, umfassend die Schritte bestehend aus:

1) Herstellen einer Emulsion von lipidreichem Mikroalgenmehl in Wasser mit einer Trockenmasse zwischen 15 und 50 Trockengew.-%, wobei das lipidreiche Mikroalgenmehl wenigstens 10 Trockengew.-% Lipide umfasst,

2) Einleiten dieser Emulsion in einen Hochdruckhomogenisator,

3) Sprühen dieser Emulsion in einen flachbödigen Sprühtrockner, der mit einer Spülvorrichtung der Sprühkammer mit Niederdruckluft in seinem unteren Teil ausgestattet ist, unter entsprechender Regulierung, so dass

a) die Temperatur der Haupttrocknungsluft zwischen 160 und 240°C beträgt,

b) die Temperatur der Luft in dem Teil der Spülvorrichtung höchstens 70°C, bevorzugt höchstens 65°C, bevorzugter zwischen 50 und 60°C, beträgt,

c) das Verhältnis zwischen dem Luftdurchsatz der Spülvorrichtung und dem Luftdurchsatz der Haupttrocknung einen Wert größer als 1/3, bevorzugt zwischen 1/3 und 1/2, beträgt,

d) die Temperatur der Kühlluft zwischen 25 und 35°C beträgt, so dass das Mehl bei Verlassen des Sprühtrockners eine Temperatur zwischen 60 und 90°C aufweist,

4) Sammeln des auf diese Weise erhaltenen Mikroalgenmehls.

**2.** Mikroalgenmehl gemäß Anspruch 1, **dadurch gekennzeichnet, dass** die Mikroalgen zur Gattung *Chlorella* gehören, bevorzugt *Chlorella protothecoides* sind.

**3.** Verfahren zur Herstellung des Mikroalgenmehls gemäß einem der Ansprüche 1 und 2, **dadurch gekennzeichnet, dass** es umfasst:

1) Herstellen einer Emulsion von lipidreichem Mikroalgenmehl in Wasser mit einer Trockenmasse zwischen 15 und 50 Trockengew.-%, wobei das lipidreiche Mikroalgenmehl wenigstens 10 Trockengew.-% Lipide umfasst,
2) Einleiten dieser Emulsion in einen Hochdruckhomogenisator,
3) Sprühen dieser Emulsion in einen flachbödigen Sprühtrockner, der mit einer Spülvorrichtung der Sprühkammer mit Niederdruckluft in seinem unteren Teil ausgestattet ist, unter entsprechender Regulierung, so dass

a) die Temperatur der Haupttrocknungsluft zwischen 160 und 240°C beträgt,
b) die Temperatur der Luft in dem Teil der Spülvorrichtung höchstens 70°C, bevorzugt höchstens 65°C, bevorzugter zwischen 50 und 60°C, beträgt,
c) das Verhältnis zwischen dem Luftdurchsatz der Spülvorrichtung und dem Luftdurchsatz der Haupttrocknung einen Wert größer als 1/3, bevorzugt zwischen 1/3 und 1/2, beträgt,
d) die Temperatur der Kühlluft zwischen 25 und 35°C beträgt, so dass das Mehl bei Verlassen des Sprühtrockners eine Temperatur zwischen 60° und 90°C aufweist,

4) Sammeln des auf diese Weise erhaltenen Mikroalgenmehls.

**4.** Verwendung des Mehls nach einem der Ansprüche 1 und 2 im Nahrungsmittelbereich.

**5.** Verfahren zur Herstellung einer Nahrungsmittelzusammensetzung umfassend Hinzufügen eines Mikroalgenmehls gemäß einem der Ansprüche 1 und 2 zu den Bestandteilen der Nahrungsmittelzusammensetzung.

**Claims**

**1.** A microalgal flour comprising at least 10% by dry weight of lipids, the size of the flour particles being between 30 and 150 μm in diameter and said flour having a compressibility, measured on a HOSOKAWA powder tester, of between 45% and 55%, **characterized in that** it has:

- a flow value, determined according to test A mentioned in the description, of between 55% and 60% by weight for the oversize at 2000 μm,
- a dispersibility and a wettability, expressed, according to test B mentioned in the description, by:

- the height of the product decanted in a beaker, having a value of between 0 and 2 mm;
- a degree of wetting having a value of more than 70%, preferably more than 80% of the total powder,

and **in that** it is prepared according to a process comprising the steps consisting of:

1) preparing an emulsion of lipid-rich microalgal flour in water with a solids content of between 15% and 50% by dry weight, the lipid-rich microalgal flour comprising at least 10% by dry weight of lipids,
2) introducing this emulsion into a high-pressure homogenizer,
3) spraying this emulsion in a flat-bottom spray dryer equipped with an air broom for sweeping the spray drying chamber with low-pressure air in its lower portion, while making adjustments to ensure that:

a) the temperature of the main drying air is between 160 and 240°C,
b) the temperature of the air in the air broom portion is at most 70°C, preferably at most 65°C, more preferably between 50 and 60°C,
c) the ratio of the flow rate of the air from the air broom to the flow rate of the main drying air has a value greater than 1/3, preferably between 1/3 and 1/2,
d) the temperature of the cooling air is between 25 and 35°C, such that the flour leaving the spray dryer has a temperature of between 60°C and 90°C,

4) collecting the microalgal flour thus obtained.

2. The microalgal flour as claimed in claim 1, **characterized in that** the microalgae are of the *Chlorella* genus, preferably *Chlorella protothecoides.*

3. A process for preparing the microalgal flour as claimed in any one of claims 1 and 2, **characterized in that** it comprises:

   1) preparing an emulsion of lipid-rich microalgal flour in water with a solids content of between 15% and 50% by dry weight, the lipid-rich microalgal flour comprising at least 10% by dry weight of lipids,
   2) introducing this emulsion into a high-pressure homogenizer,
   3) spraying this emulsion in a flat-bottom spray dryer equipped with an air broom for sweeping the spray drying chamber with low-pressure air in its lower portion, while making adjustments to ensure that:

   a) the temperature of the main drying air is between 160 and 240°C,
   b) the temperature of the air in the air broom portion is at most 70°C, preferably at most 65°C, more preferably between 50 and 60°C,
   c) the ratio of the flow rate of the air from the air broom to the flow rate of the main drying air has a value greater than 1/3, preferably between 1/3 and 1/2,
   d) the temperature of the cooling air is between 25 and 35°C, such that the flour leaving the spray dryer has a temperature of between 60°C and 90°C,

   4) collecting the microalgal flour thus obtained.

4. The use of the flour of any one of claims 1 and 2 in the food sectors.

5. A method for preparing a food composition comprising the addition of a microalgal flour as claimed in any one of claims 1 and 2 to ingredients of the food composition.

EP 3 027 053 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6607900 B
- US 6372460 B
- US 6255505 B
- US 5330913 A
- US 2011256282 A
- WO 2010045368 A
- WO 2010120923 A
- US 20100297296 A

**Littérature non-brevet citée dans la description**

- **DE K. MASTERS.** Spray Drying Handbook. British Library ou à la Library of Congress, 1991
- BETE® Spray Dry Manual. 2005
- **HAUGAARD SORENSEN et al.** Méthodes d'analyse des produits laitiers déshydratés. 1978
- **CAYOT ; LORIENT.** Structures et technofonctions des protéines du lait. Airlait Recherches: Tec et Doc, 1998